(19) Europäisches Patentamt
European Patent Office
Office européen des brevets

(11) Publication number : **0 361 156 B1**

(12) **EUROPEAN PATENT SPECIFICATION**

(45) Date of publication of patent specification : **10.03.93 Bulletin 93/10**

(51) Int. Cl.$^5$ : **A61K 31/44,** // (A61K31/44, 31:34, 31:21)

(21) Application number : **89116468.3**

(22) Date of filing : **06.09.89**

(54) **Pharmaceuticals.**

(30) Priority : **20.09.88 DE 3831949**

(43) Date of publication of application : **04.04.90 Bulletin 90/14**

(45) Publication of the grant of the patent : **10.03.93 Bulletin 93/10**

(84) Designated Contracting States : **AT BE CH DE ES FR GB GR IT LI LU NL SE**

(56) References cited :
**EP-A- 0 114 270**
**EP-A- 0 207 397**
**FR-A- 2 454 807**

(56) References cited :
**Bisosis 0017551039 33029544 RRM: M. LEITOLD, W. FLEISSIG: "Comparative investigation of different classes of compounds in experimental myocardial infarction in the rat" & 28th Spring meeting of the deutsche Gesellschaft fuer Pharmakologie und Toxikologie, March 10-13, 1987, Naunyn-Schmiedeberg's Arch Pharmacol. 335(suppl.) 1987, R65**

(73) Proprietor : **Heinrich Mack Nachf.**
**Postfach 2064**
**W-7918 Illertissen (DE)**

(72) Inventor : **Stoss, Peter, Dr.**
**Mozartstrasse 15**
**W-7918 Illertissen (DE)**
Inventor : **Leitold, Matyas, Dr.**
**Klauflügelweg 21**
**W-7950 Biberach (DE)**

(74) Representative : **Lederer, Franz, Dr. et al**
**Lederer, Keller & Riederer Patentanwälte**
**Lucile-Grahn-Strasse 22**
**W-8000 München 80 (DE)**

EP 0 361 156 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a pharmaceutical for treating cardiovascular diseases, especially for preventing and treating myocardial infarct.

German Offenlegungsschrift 3,248,548 (= EP-A 114,270) discloses new acyl derivatives of 1,4:3,6-dianhydrohexitols which can be used as peripheral and central vasodilators and as coronary therapeutics. These compounds are dihydropyridylcarbonyl derivatives.

Nitro derivatives such as isosorbide 5-mononitrate (5-ISM), isosorbide 2-mononitrate (2-ISM), glycerol trinitrate (GTN), glycerol 1-mononitrate (1-GMN) and glycerol 2-mononitrate (2-GMN) have long been known as vasodilating and coronary therapeutic pharmaceuticals.

It has now been found that the specific above-mentioned acyl derivatives of 1,4:3,6-dianhydrohexitols exhibit, when combined with the coronary therapeutic nitro derivatives, surprising effects which have not hitherto been observed and are extremely therapeutically important and desired, and, especially, significantly reduce the myocardial infarct rate.

German Offenlegungsschrift 3,523,544 (= EP-A-207,398) discloses a solid combination product which contains nifedipine and isosorbide 5-mononitrate (5-ISM) and can be used for the long-term treatment of heart diseases, especially of diseases of the coronaries and of the myocardium. There is no mention therein of a reduction in the myocardial infarct rate.

German Offenlegungsschrift 3,523,540 (= EP-A-207,397) discloses combination products of certain dihydropyridines with 5-ISM, which are likewise suitable for controlling heart diseases. Nisoldipine and nitrendipine are disclosed therein as preferred dihydropyridines. These known combinations are said to prevent the development of tolerance to the nitrate component and to reduce undesired side effects. An intentional reduction in the myocardial infarct rate is not disclosed.

Although the acyl derivatives used in the combination products according to the invention also contain the dihydropyridyl radical, their structures differ considerably from nifedipine on the one hand and the dihydropyridines such as nisoldipine and nitrendipine on the other hand.

The invention relates to pharmaceuticals consisting of

a) a compound of the general formula I

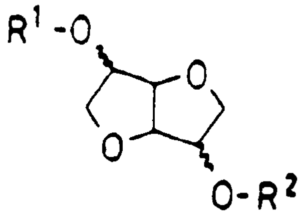

I

in which

$R^1$ represents hydrogen, a lower acyl radical having 2 to 5 carbon atoms, a pyridylcarbonyl radical or $NO_2$, and

$R^2$ represents a 1,4-dihydropyridylcarbonyl radical of the general formula II

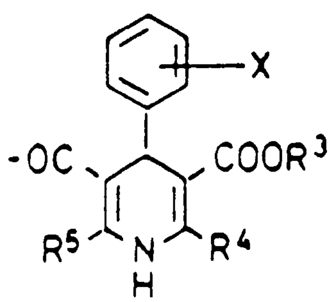

II

in which

X represents hydrogen or 1, 2 or 3 identical or different substituents from the group comprising alkoxy, alkyl, cyano, dialkylamino, halogen, nitro or trifluoromethyl, or represents a methylenedioxy group,

$R^3$ denotes a straight-chain or branched, saturated or unsaturated hydrocarbon radical having 1 to 5 carbon atoms, it being possible for the chain optionally to be interrupted by an oxygen atom and/or for

the hydrocarbon radical optionally to be substituted by a cyano group, and

$R^4$ and $R^5$ are identical or different and each represents a lower alkyl group,

or a pharmaceutically acceptable salt of a compound of this type, and

b) isosorbide 5-mononitrate (5-ISM) or isosorbide 2-mononitrate (2-ISM) or glycerol trinitrate (GTN) or glycerol 1-mononitrate (1-GMN) or glycerol 2-mononitrate (2-GMN), plus optionally,

c) customary inactive ingredients and vehicles.

The invention also embraces the process for the preparation of these pharmaceuticals, which consists in that a) a compound of the general formula I according to Claim 1 is converted with b) 5-ISM or 2-ISM or GTN or 1-GMN or 2-GMN in a ratio by weight of a:b of 8:1 to 1:80, preferably 3:1 to 1:8, by use of c) customary inactive ingredients and vehicles, into a suitable, preferably solid, administration form.

The invention furthermore relates to the use of a) a compound of the general formula I together with b) 5-ISM or 2-ISM or GTN or 1-GMN or 2-GMN for the preparation of pharmaceuticals for preventing and treating cardiovascular diseases, especially angina pectoris and myocardial infarct.

The preparation of the compounds of the general formula I is described in German Offenlegungsschrift 3,248,548.

The substituents -$OR^1$ and $OR^2$ in the compounds of the general formula I can be both exo- and endo-linked to the ring system. This is expressed in the formula I by a wavy line (~~), thus including derivatives both of isomannide (endo/endo), and of isosorbide (endo/exo) and of isoidide (exo/exo).

Cations for pharmaceutically acceptable salts embrace sodium, potassium, lithium, calcium, magnesium, ammonium and organic amines.

The pharmaceutical according to the invention can be administered once or more than once a day in a dosage of 1-400 mg based on the compound of the formula I per dose. However, the dosage should be established in the individual case as a function of the severity of the disease, the weight and constitution of the patient as well as other relevant factors, by the administering physician. Those combinations which contain 5-40 mg of the compounds of the general formula I and 10-40 mg of 5-ISM or 10-40 mg of 2-ISM or 5-20 mg of GTN or 20-100 mg of 1-GMN or 50-400 mg of 2-GMN are of particular interest.

Pharmaceuticals are to be understood to be those which contain the active substance or the active substance combination, where appropriate with customary inactive ingredients and vehicles, in a form which can be administered to the patients. Particularly preferred in the present case are solid and liquid pharmaceutical formulations which can be administered orally.

The active substances according to the invention can be administered alone, but as a rule they are administered mixed with a suitable pharmaceutical excipient or diluent, which is selected taking account of the route of administration. The active substances are preferably administered orally, for example in the form of tablets containing vehicles such as, for example, starch or lactose, or in capsules in the form of solutions or suspensions in customary pharmaceutical inactive ingredients which can additionally contain flavourings or colourings. On administration to animals, the active substances are normally added to the animal feed or the drinking water.

The pharmaceuticals according to the invention are outstandingly suitable for treating cardiovascular diseases of a wide variety of types. It is true that the individual components, namely both the compounds of the general formula I as well as 5-ISM, 2-ISM, GTN, 1-GMN and 2-GMN, are also known to act in this area. However, an unexpected advance is achieved with the new active substance combinations in that new types of action which are not intrinsic to the individual components appear, and desired properties are enhanced and undesired are suppressed, and thus, on the one hand, an alteration in the spectrum of action and, on the other hand, a synergistic effect of very great therapeutic significance is achieved.

It has emerged, in particular, that it is possible, surprisingly, to bring about a distinct reduction in the myocardial infarct rate with the pharmaceuticals according to the invention. Hence, these combinations can be used as pharmaceuticals for the prophylaxis and therapy of myocardial infarct. This reveals a new use for preventing and treating angina pectoris, where it has not hitherto been possible to employ calcium antagonists of the 1,4-dihydropyridine type because an action is lacking.

In addition to the synergistic effect and the new action preventing myocardial infarct, the compounds of the general formula I are distinguished by favourable toxicity data. Thus, compound A which is used in the example is distinctly superior to nifedipine, as is evident from Table 2 hereinafter.

Owing to distinctly lower toxicity and the synergism, the active substance combinations according to the invention are also especially suitable for the long-term treatment of the abovementioned diseases. Furthermore, they bring about a lowering both of the preload and of the afterload on the heart, which likewise represents a great advantage and a significant advance in therapy. Another surprising, but extremely desirable, property of the pharmaceutical combinations according to the invention is that an increase in heart rate hardly ever occurs. In this respect there is a distinct superiority to, for example, nifedipine and nitrendipine, where

this undesired side effect is distinctly present. Moreover, a distinct prolongation of the duration of action is achieved with the combinations according to the invention by comparison with the individual substances.

In order to demonstrate the therapeutic effect, the results of some pharmacological tests are listed in Table 1 hereinafter. In this, (-)-5-[1,4-dihydro-2,6-dimethyl-3-methoxycarbonyl-4-(3-nitrophenyl)-5-pyridylcarbonyl]isosorbide is used as an example of a compound of the general formula I (called compound A):

TABLE 1

Effect of various substances on the survival rate of rats at various times after ligature of the left coronary artery. Oral administration of the substance 5 minutes before the coronary ligature.

| Substance | Oral dose mg/kg | No. of surviving rats 1 h | 24 h | 7 d |
|---|---|---|---|---|
| Control | 0.5 ml/100 g rat 1 % CMC | 28/98 | 26/98 | 22/98 |
| 5-ISM | 200 | 13/20* | 13/20* | 13/20* |
| GTN | 200 | 14/20* | 14/20* | 14/20* |
| Compound A | 18.75 | 17/20* | 16/20* | 15/20* |
| 5-ISM + Compound A + | 200 18.75 | 20/20*** | 20/20*** | 19/20*** |
| GTN + Compound A/+ | 200 18.75 | 20/20*** | 20/20*** | 19/20*** |

Significance compared with controls: $p < 0.05$*
$p < 0.001$***

CMC = carboxymethylcellulose

TABLE 2

Acute toxicity after a single oral administration

| Substance | Species | $LD_{50}$ mg/kg |
|---|---|---|
| Compound A | mouse | 1250 |
| Nifedipine* | mouse | 494 |
| Compound A | rat | >4000 |
| Nifedipine* | rat | 1022 |

* value from the literature

Example 1

10 g of a compound of the general formula I are mixed with 10 g of 5-ISM (50 % in lactose), a further 20 g of lactose, and 25 g of maize starch and 15 g of microcrystalline cellulose and granulated as usual. The resulting granules are compressed to give tablets of various sizes, and thus various contents of active substance, or are used to fill gelatin capsules of various capacities, and thus different contents of active substance.

Example 2

15 g of a compound of the general formula I are mixed with 100 g of a 5 per cent trituration of GTN in lactose and 50 g of maize starch and granulated as familiar to all those skilled in the art. The dried and screened granules are mixed with 10 g of sodium stearyl fumarate and 50 g of talc and compressed to form tablets of various sizes or used to fill gelatin capsules of various sizes.

**Claims**

**1.** Pharmaceuticals consisting of

a) a compound of the general formula I

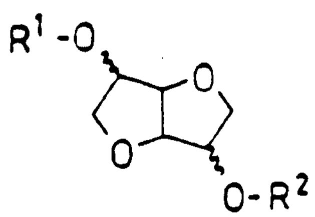

I

in which

$R^1$ represents hydrogen, a lower acyl radical having 2 to 5 carbon atoms, a pyridylcarbonyl radical or $NO_2$, and

$R^2$ represents a 1,4-dihydropyridylcarbonyl radical of the general formula II

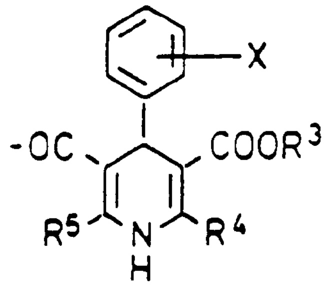

II

in which

X represents hydrogen or 1, 2 or 3 identical or different substituents from the group comprising alkoxy, alkyl, cyano, dialkylamino, halogen, nitro or trifluoromethyl, or represents a methylenedioxy group,

$R^3$ denotes a straight-chain or branched, saturated or unsaturated hydrocarbon radical having 1 to 5 carbon atoms, it being possible for the chain optionally to be interrupted by an oxygen atom and/or for the hydrocarbon radical optionally to be substituted by a cyano group, and

$R^4$ and $R^5$ are identical or different and each represents a lower alkyl group,

or a pharmaceutically acceptable salt of a compound of this type, and

b) isosorbide 5-mononitrate (5-ISM) or isosorbide 2-mononitrate (2-ISM) or glycerol trinitrate (GTN) or glycerol 1-mononitrate (1-GMN) or glycerol 2-mononitrate (2-GMN), plus optionally,

c) customary inactive ingredients and vehicles.

2. Pharmaceutical according to Claim 1, characterized in that it contains the components a) and b) in a ratio by weight of 8:1 to 1:80, preferably 3:1 to 1:8.

3. Pharmaceutical according to Claim 1 or 2, characterized in that it is in a solid or liquid form which can be administered orally.

4. Pharmaceutical according to Claim 3, characterized in that one dose unit contains 1-400, preferably 5-40, mg of component a).

5. Pharmaceutical according to one of Claims 1-4, characterized in that it contains 5-40 mg of the compound of the general formula I per 10-40 mg of 5-ISM or 10-40 mg of 2-ISM or 5-20 mg of GTN or 20-100 mg of 1-GMN or 50-400 mg of 2-GMN.

6. Pharmaceutical according to one of Claims 1-5, characterized in that component a) is a compound of the general formula I in which $R^1$ denotes hydrogen or an $NO_2$ group.

7. Pharmaceutical according to one of Claims 1-5, characterized in that component a) is (-)-5-[1,4-dihydro-2,6-dimethyl-3-methoxycarbonyl-4-(3-nitrophenyl)-5-pyridylcarbonyl]isosorbide.

8. Process for the preparation of a pharmaceutical, characterized in that a) a compound of the general formula I according to Claim 1 is converted with b) 5-ISM or 2-ISM or GTN or 1-GMN or 2-GMN in a ratio by weight of a:b of 8:1 to 1:80, preferably 3:1 to 1:8, by use of c) customary inactive ingredients and vehicles, into a suitable, preferably solid, administration form.

9. Use of a) a compound of the general formula I according to Claim 1, together with b) 5-ISM or 2-ISM or GTN or 1-GMN or 2-GMN for the preparation of pharmaceuticals for treating cardiovascular diseases, especially for the prophylaxis and therapy of angina pectoris and myocardial infarct.

**Patentansprüche**

1. Pharmazeutika bestehend aus

a) einer Verbindung der allgemeinen Formel (I)

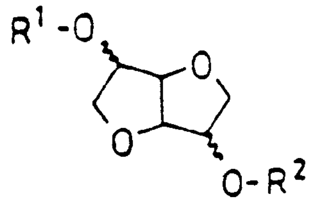

(I),

worin $R^1$ Wasserstoff, einen niederen Acylrest mit 2 bis 5 Kohlenstoffatomen, einen Pyridylcarbonylrest oder $NO_2$ bedeutet und $R^2$ einen 1,4-Dihydropyridylcarbonylrest der allgemeinen Formel (II)

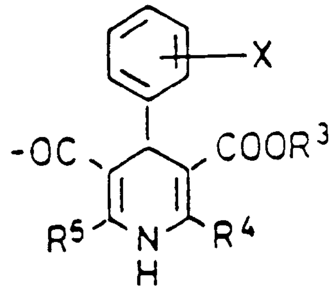

(II)

darstellt, in dem X Wasserstoff oder 1, 2 oder 3 gleiche oder verschiedene Substituenten aus der Gruppe umfassend Alkoxy, Alkyl, Cyano, Dialkylamino, Halogen, Nitro oder Trifluormethyl bedeutet oder eine Methylendioxygruppe ist, $R^3$ einen geradkettigen oder verzweigten, gesättigten oder ungesättigten Kohlenwasserstoffrest mit 1 bis 5 Kohlenstoffatomen darstellt, wobei es möglich ist, daß die Kette gegebenenfalls durch ein Sauerstoffatom unterbrochen ist und/oder der Kohlenwasserstoffrest gegebenenfalls durch eine Cyanogruppe substituiert ist, und $R^4$ und $R^5$ gleich oder verschieden sind und jeweils eine nied.Alkylgruppe bedeuten, oder einem pharmazeutisch annehmbaren Salz einer Verbindung dieses Typs und
b) Isosorbid-5-mononitrat (5-ISM), Isosorbid-2-mononitrat (2-ISM), Glycerintrinitrat (GTN), Glycerin-1-mononitrat (1-GMN) oder Glycerin-2-mononitrat (2-GMN) plus gegebenenfalls
c) üblichen inaktiven Bestandteilen und Trägern.

2. Pharmazeutikum nach Anspruch 1, dadurch gekennzeichnet, daß es die Bestandteile a) und b) in einem Gewichtsverhältnis von 8:1 bis 1:80, vorzugsweise 3:1 bis 1:8, enthält.

3. Pharmazeutikum nach Anspruch 1 oder 2, dadurch gekennzeichnet, daß es in einer festen oder flüssigen Form ist, die oral verabreicht werden kann.

4. Pharmazeutikum nach Anspruch 3, dadurch gekennzeichnet, daß eine Dosiseinheit 1 bis 400, vorzugsweise 5 bis 40 mg Bestandteil a) enthält.

5. Pharmazeutikum nach einem der Ansprüche 1 bis 4, dadurch gekennzeichnet, daß es 5 bis 40 mg der Verbindung der allgemeinen Formel (I) pro 10 bis 40 mg 5-ISM, 10 bis 40 mg 2-ISM, 5 bis 20 mg GTN, 20 bis 100 mg 1-GMN oder 50 bis 400 mg 2-GMN enthält.

6. Pharmazeutikum nach einem der Ansprüche 1 bis 5, dadurch gekennzeichnet, daß der Bestandteil a) eine Verbindung der allgemeinen Formel (I) ist, worin $R^1$ Wasserstoff oder eine Gruppe $NO_2$ bedeutet.

7. Pharmazeutikum nach einem der Ansprüche 1 bis 5 dadurch gekennzeichnet, daß der Bestandteil a) (-)-5-[1,4-Dihydro-2,6-dimethyl-3-methoxycarbonyl-4-(3-nitrophenyl)-5-pyridylcarbonyl]-isosorbid ist.

8. Verfahren zur Herstellung eines Pharmazeutikums, dadurch gekennzeichnet, daß a) eine Verbindung der allgemeinen Formel (I) nach Anspruch 1 mit b) 5-ISM, 2-ISM, GTN, 1-GMN oder 2-GMN in einem Gewichtsverhältnis von a:b von 8:1 bis 1:80, vorzugsweise 3:1 bis 1:8, unter Verwendung c) üblicher inaktiver Bestandteile und Träger in eine geeignete, vorzugsweise feste, Verabreichungsform überführt wird.

9. Verwendung a) einer Verbindung der allgemeinen Formel (I) nach Anspruch 1 zusammen mit b) 5-ISM, 2-ISM, GTN, 1-GMN oder 2-GMN zur Herstellung von Pharmazeutika für die Behandlung von

kardiovaskulären Krankheiten, insbesondere für die Prophylaxe und Therapie von Angina pectoris und Myokardinfarkt.

## Revendications

1. Agents pharmaceutique consistant en
   a) un composé de formule générale I

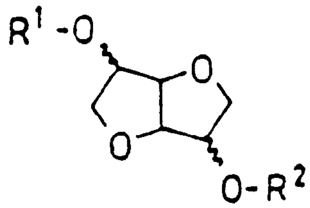

I

dans laquelle

$R^1$ représente l'hydrogène, un radical acyle inférieur ayant 2 à 5 atomes de carbone, un radical pyridylcarbonyle ou $NO_2$, et

$R^2$ représente un radical 1,4-dihydropyridylcarbonyle de formule générale II

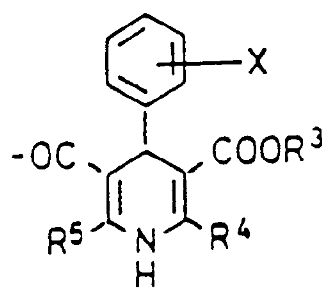

II

dans laquelle

X représente l'hydrogène ou bien 1, 2 ou 3 substituants identiques ou différents choisis dans le groupe comprenant des radicaux alkoxy, alkyle, cyano, dialkylamino, halogéno, nitro et trifluorométhyle, ou bien représente un groupe méthylènedioxy,

$R^3$ représente un radical hydrocarboné saturé ou insaturé, à chaîne droite ou ramifié, ayant 1 à 5 atomes de carbone, la chaîne pouvant être facultativement interrompue par un atome d'oxygène et/ou le radical hydrocarboné pouvant être facultativement substitué avec un groupe cyano, et

$R^4$ et $R^5$ sont identiques ou différents et représentent chacun un groupe alkyle inférieur,

ou un sel pharmaceutiquement acceptable d'un composé de ce type, et

b) 5-mononitrate d'isosorbide (5-ISM) ou 2-mononitrate d'isosorbide (2-ISM) ou trinitrate de glycérol (GTN) ou 1-mononitrate de glycérol (1-GMN) ou 2-mononitrate de glycérol (2-GMN), plus, facultativement,

c) des ingrédients et véhicules inactifs usuels.

2. Agent pharmaceutique suivant la revendication 1, caractérisé en ce qu'il contient les constituants a) et b) en un rapport pondéral de 8:1 à 1:80, de préférence de 3:1 à 1:8.

3. Agent pharmaceutique suivant la revendication 1 ou 2, caractérisé en ce qu'il est sous une forme solide ou liquide qui peut être administrée par voie orale.

4. Agent pharmaceutique suivant la revendication 3, caractérisé en ce qu'une dose unitaire contient 1 à 400, de préférence 5 à 40, mg de constituant a).

5. Agent pharmaceutique suivant l'une des revendications 1 à 4, caractérisé en ce qu'il contient 5 à 40 mg du composé de formule générale I pour 10 à 40 mg de 5-ISM ou 10 à 40 mg de 2-ISM ou 5 à 20 mg de GTN ou 20 à 100 mg de 1-GMN ou bien 50 à 400 mg de 2-GMN.

**6.** Agent pharmaceutique suivant l'une des revendications 1 à 5, caractérisé en ce que le constituant a) est un composé de formule générale I dans laquelle $R^1$ représente l'hydrogène ou un groupe $NO_2$.

**7.** Agent pharmaceutique suivant l'une des revendications 1 à 5, caractérisé en ce que le constituant a) est le (-)-5-[1,4-dihydro-2,6-diméthyl-3-méthoxycarbonyl-4-(3-nitrophényl)-5-pyridylcarbonyl]isosorbide.

**8.** Procédé de préparation d'un agent pharmaceutique, caractérisé en ce que a) un composé de formule générale I suivant la revendication 1 est transformé avec b) du 5-ISM ou du 2-ISM ou du GTN ou du 1-GMN ou du 2-GMN en un rapport pondéral a:b de 8:1 à 1:80, de préférence de 3:1 à 1:8, au moyen c) d'ingrédients et de véhicules inactifs usuels, en une forme convenable d'administration, de préférence solide.

**9.** Utilisation a) d'un composé de formule générale I suivant la revendication 1, en association avec b) du 5-ISM ou du 2-ISM ou du GTN ou du 1-GMN ou bien du 2-GMN pour la préparation d'agents pharmaceutiques destinés au traitement de maladies cardiovasculaires, notamment à la prophylaxie et la thérapeutique de l'angine de poitrine et de l'infarctus du myocarde.